# EUROPÄISCHE PATENTSCHRIFT

(11) **EP 0 749 970 B1**
(45) Veröffentlichungstag und Bekanntmachung des Hinweises auf die Patenterteilung: **17.01.2001**
(21) Anmeldenummer: 96109134.5
(22) Anmeldetag: 07.06.1996
(51) Int. Cl.: C07D 417/12, C07D 493/04, A61K 31/425

(54) **Benzisothiazolyl-substituierte Aminomethylchromane**
Benzisothiazolyl-substituted aminomethylchroman derivatives
Dérivés d'aminométhylchromanes substitués par un groupe benzisothiazolyle

(30) Priorität: 19.06.1995 DE 19522088
(43) Veröffentlichungstag der Anmeldung: 27.12.1996
(73) Patentinhaber: BAYER AG, 51368 Leverkusen (DE)
(72) Erfinder: Schohe-Loop, Rudolf, Dr., 42327 Wuppertal (DE); Heine, Hans-Georg, Dr., 47800 Krefeld (DE); Seidel, Peter-Rudolf, Dr., 51147 Köln (DE); Kanhai, Wolfgang, Dr., 42115 Wuppertal (DE); Schuhmacher, Joachim, Dr., 42113 Wuppertal (DE); Friedl, Arno, Dr., 51427 Bergisch Gladbach (DE); Horvath, Ervin, Dr., 51373 Leverkusen (DE); Glaser, Thomas, Dr., 51491 Overath (DE); Jork, Reinhard, Prof. Dr., 51491 Overath (DE)

(56) Entgegenhaltungen:
- EP-A- 0 352 613
- EP-A- 0 540 914

## Beschreibung

Die vorliegende Erfindung betrifft Benzisothiazolyl-substituierte Aminomethylchromane, Verfahren zu ihrer Herstellung und ihre Verwendung in Arzneimitteln, insbesondere als Mittel zur Bekämpfung von Erkrankungen des zentralen Nervensystems.

Aus den Publikationen EP 352 613 und EP 540 914 sind Aminotetralin- und- -chromanderivate mit ZNS-Aktivität bekannt.

Die Erfindung betrifft neue Benzisothiazolyl-substituierte Aminomethylchromane der allgemeinen Formel (I), 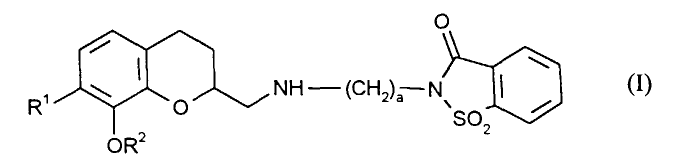 in welcher
- R¹: für Wasserstoff steht,
und
- R²: für einen Rest der Formel -CH(CH₃)₂ oder -CH₂-C(CH₃)₂-Cl steht,
oder
- R¹ und R²: gemeinsam einen Rest der Formel 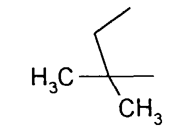 bilden,
und
- a: für eine Zahl 3, 4 oder 5 steht,
gegebenenfalls in einer isomeren Form und deren Salze.

Überraschenderweise zeigen die erfindungsgemäßen Verbindungen eine gute Wirksamkeit bei der Behandlung von Schädigungen infolge cerebraler Infarkte.

Im Rahmen der vorliegenden Erfindung werden physiologisch unbedenkliche Salze bevorzugt. Physiologisch unbedenkliche Salze der Benzisothiazolyl-substituierten Aminomethylchromane können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Im Rahmen der vorliegenden Erfindung können die erfindungsgemäßen Verbindungen in verschiedenen stereoisomeren Formen vorliegen. Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Beispielsweise seien folgende Isomere genannt:

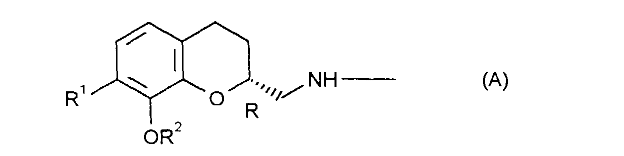

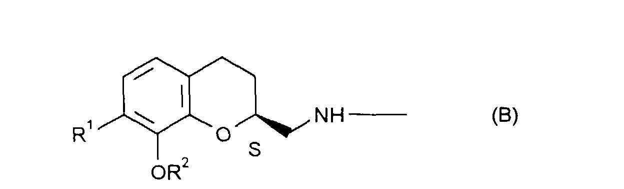

Bevorzugt sind Verbindungen der allgemeinen Formel (I),
in welcher
- R¹: für Wasserstoff steht,
und
- R²: für einen Rest der Formel -CH(CH₃)₂ oder -CH₂-C(CH₃)₂-Cl steht,
oder
- R¹ und R²: gemeinsam einen Rest der Formel 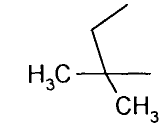 bilden,
und
- a: für eine Zahl 3 oder 4 steht,
gegebenenfalls in einer isomeren Form und deren Salze.

Besonders bevorzugt sind Verbindungen der allgemeinen Formel (I),
in welcher
- R¹: für Wasserstoff steht,
und
- R²: für einen Rest der Formel -CH(CH₃)₂ oder -CH₂-C(CH₃)₂-Cl steht,
oder
- R¹ und R²: gemeinsam einen Rest der Formel 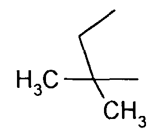 bilden,
und
- a: für die Zahl 4 steht,
gegebenenfalls in einer isomeren Form und deren Salze.

Die erfindungsgemäßen Benzisothiazolyl-substituierten Aminomethylchromane werden hergestellt, indem man
[A] Amine der allgemeinen Formel (II) 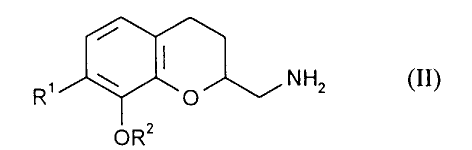 in welcher
   - R¹ und R²: die oben angegebene Bedeutung haben,
   mit Verbindungen der allgemeinen Formel (III) 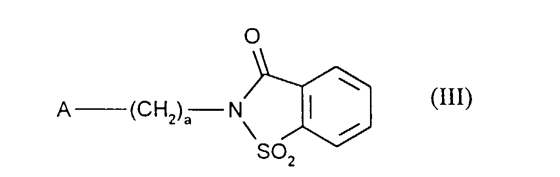 in welcher
   - A: für eine typische Abgangsgruppe wie Chlor, Brom, Iod, Tosylat, Mesylat oder für die Gruppe -OSO₂CF₃ steht,
   und
   - a: die oben angegebene Bedeutung hat,
   in inerten Lösemitteln, gegebenenfalls in Anwesenheit einer Base umsetzt, oder
[B] Verbindungen der allgemeinen Formel (IV) 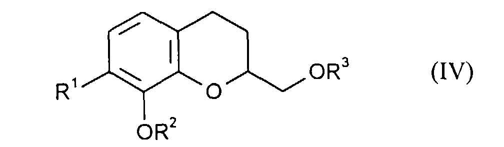 in welcher
   - R' und R²: die oben angegebene Bedeutung haben
   und
   - R³: für einen Rest der Formel -SO₂CF₃, -SO₂CH₃ oder Tosylat steht,
   mit Aminen der allgemeinen Formel (V) 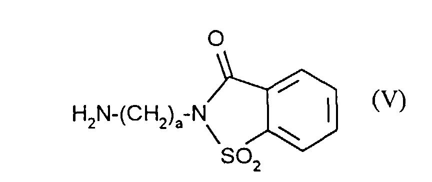 in welcher
   - a: die oben angegebene Bedeutung hat,
   in inerten Lösemitteln, gegebenenfalls in Anwesenheit einer Base und/oder eines Katalysators umsetzt,
   oder
[C] Verbindungen der allgemeinen Formel (VI) 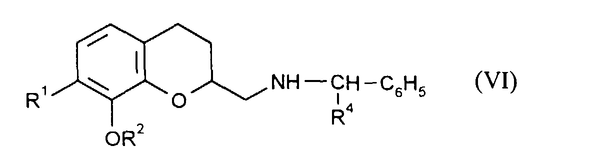 in welcher
   - R¹ und R²: die oben angegebene Bedeutung haben,
   und
   - R⁴: für Wasserstoff oder Methyl steht,
   zunächst entweder mit Formaldehyd oder Formaldehydderivaten und Verbindungen der allgemeinen Formel (VII) 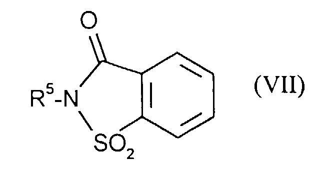 in welcher
   - R⁵: für einen Rest der Formel

   HC≡C-(CH₂)_{b}-

   steht,
   worin
   - b: eine Zahl 0, 1 oder 2 bedeutet,
   in einer Mannich-analogen Reaktion umsetzt und dann hydriert.

Die erfindungsgemäßen Verfahren können durch folgendes Formelschema beispielhaft erläutert werden:

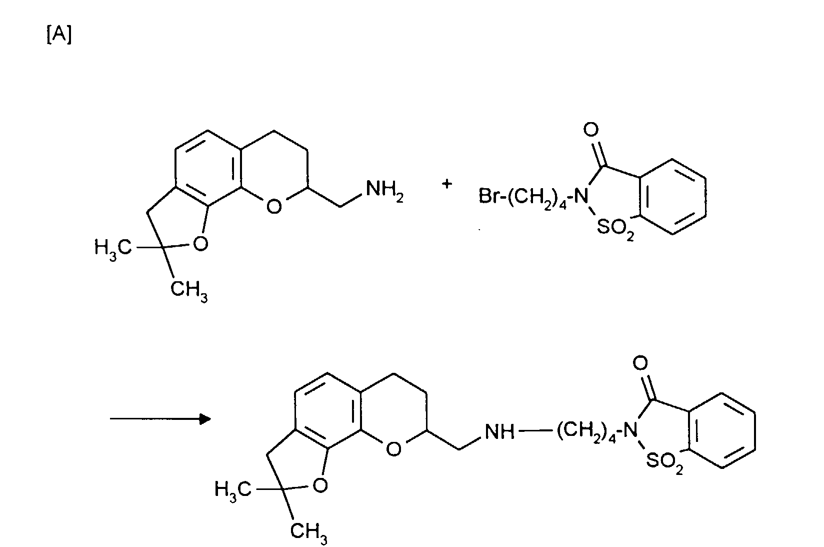

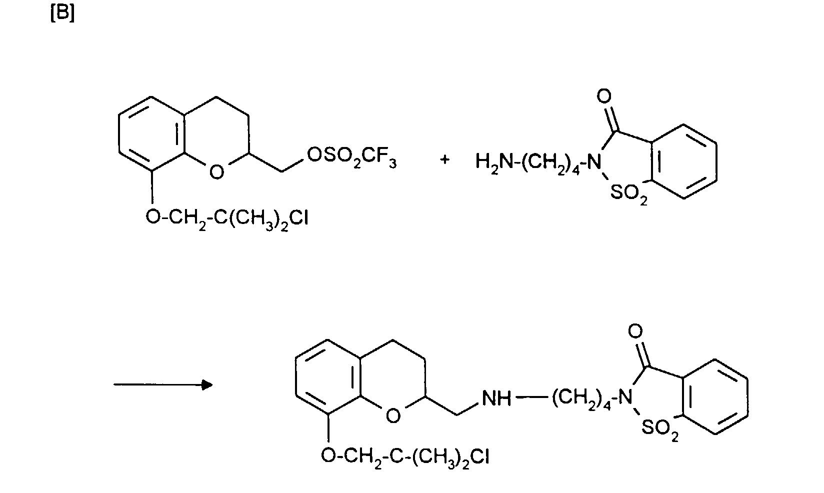

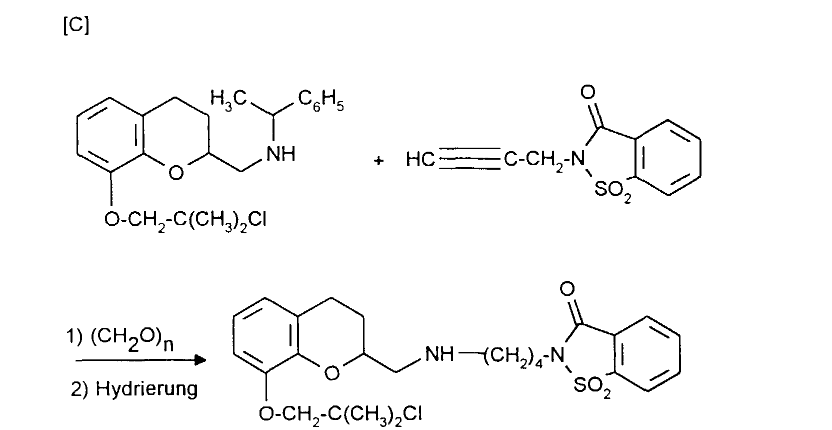

Als Lösemittel für die Verfahren [A] und [B] eignen sich die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Butylmethylether, oder Ketone wie Aceton oder Butanon, oder Amide wie Dimethylformamid oder Hexamethylphosphorsäuretriamid, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden. Bevorzugt sind Methanol, Ethanol, Isopropanol, Dimethylformamid und Acetonitril.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Pyridin, Picolin oder N-Methylpiperidin, oder Amide wie Natriumamid oder Lithiumdiisopropylamid, oder metallorganische Verbindungen wie Butyllithium oder Phenyllithium. Bevorzugt sind Natrium- und Kaliumcarbonat, Pyridin und Triethylamin.

Die Umsetzungen [A] und [B] erfolgen im allgemeinen in einem Temperaturbereich von -20°C bis zur Rückflußtemperatur des Lösemittels, bevorzugt von +20°C bis zur Rückflußtemperatur des Lösemittels.

Die Verfahren [A] und [B] werden im allgemeinen bei Normaldruck durchgeführt. Es ist jedoch ebenso möglich, die Reaktion bei erhöhtem oder erniedrigtem Druck durchzuführen.

Als Reaktionsbeschleuniger werden im allgemeinen Alkaliiodide eingesetzt, bevorzugt ist Natriumiodid oder Kaliumiodid.

Die Base wird hierbei in einer Menge von 1 bis 5, bevorzugt von 1 bis 2 Mol, bezogen auf 1 Mol der Verbindungen der allgemeinen Formeln (II) und (V) eingesetzt.

Die Umsetzung mit Formaldehyd und Acetylenderivaten in einer Mannichähnlichen Reaktion wird im allgemeinen in einem der oben aufgeführten organischen Lösemittel, die sich unter den jeweiligen Reaktionsbedingungen nicht verändern, wie beispielsweise Alkohole, Ether, Kohlenwasserstoffe, Halogenkohlenwasserstoffe und Dimethylformamid sowie deren Gemische durchgeführt. Bevorzugt sind Tetrahydrofuran und 1,4-Dioxan.

Als Katalysatoren werden im allgemeinen Kupfersalze eingesetzt. Bevorzugt wird Kupfer(II)acetat. Als Formaldehydquelle gelangen Paraformaldehyd, Trioxan, Formalinlösung und gasförmiges Formaldehyd zum Einsatz. Bevorzugt wird Paraformaldehyd.

Die Reaktion erfolgt im allgemeinen in einem Temperaturbereich von 0°C bis zur Rückflußtemperatur, bevorzugt von +20°C bis +70°C.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Hydrierung (Verfahren C) erfolgt im allgemeinen mit Wasserstoff in Wasser oder einem der oben aufgeführten Lösemittel, vorzugsweise Wasser, Methanol, Ethanol, Diethylether, Tetrahydrofuran in Gegenwart von Mineralsäuren wie beispielsweise Salzsäure. Als Katalysatoren eignen sich Katalysatoren, wie beispielsweise Raney-Nickel, Palladium, Palladium auf Tierkohle oder Platin, vorzugsweise Palladium und Palladium auf Tierkohle.

Der Katalysator wird in einer Menge von 0,01 Mol bis 0,2 Mol, bevorzugt von 0,05 Mol bis 0,15 Mol jeweils bezogen auf die blockierten Verbindungen der allgemeinen Formel (IIa) eingesetzt.

Die Reaktion kann bei normalem, erhöhtem oder bei erniedrigtem Druck durchgeführt werden (z.B. 0,5 bis 25 bar). Im allgemeinen arbeitet man bei Normaldruck bis 3 bar.

Die Verbindungen der allgemeinen Formel (II) sind neu und können beispielsweise hergestellt werden, indem man

Verbindungen der allgemeinen Formel (VIII) 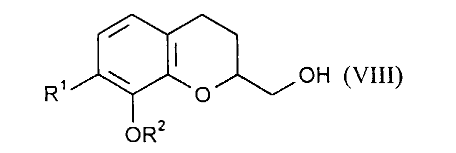 in welcher
- R¹ und R²: die oben angegebene Bedeutung haben,
zunächst mit Phthalimid in Anwesenheit von Triphenylphosphan / Azodicarbonsäurediethylester in einem der oben aufgeführten Lösemitteln, vorzugsweise Tetrahydrofuran in die Verbindungen der allgemeinen Formel (IX) 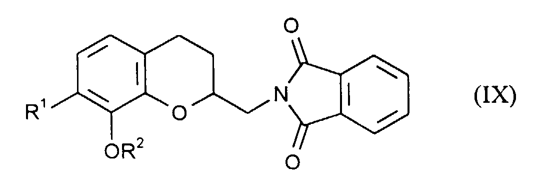 in welcher
- R¹ und R²: die oben angegebene Bedeutung haben,
überführt,
und in einem zweiten Schritt die Phthalimidgruppe in Aminoethanol abspaltet.

Die verschiedenen Reaktionsschritte verlaufen in einem Temperaturbereich von 0°C bis +100°C, vorzugsweise von Raumtemperatur bis +80°C und bei Normaldruck.

Die Verbindungen der allgemeinen Formel (IX) sind neu und können beispielsweise wie oben beschrieben hergestellt werden.

Die Verbindungen der allgemeinen Formel (VIII) sind neu und können beispielsweise hergestellt werden, indem man 8-Hydroxy-2-hydroxymethylchroman der Formel (X) 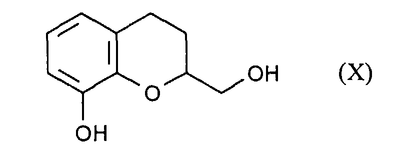 mit Verbindungen der allgemeinen Formel (XI)

R⁶-Y (XI)

in welcher
- R⁶: geradkettiges oder verzweigtes Alkyl oder Alkenyl mit bis zu 4 Kohlenstoffatomen bedeutet,
und
- Y: für Halogen, vorzugsweise für Chlor, Brom oder Jod steht,
alkyliert und gegebenenfalls in einem zweiten Schritt nach üblichen Methoden die oben unter R¹ und R² aufgeführten Reste durch Derivatisierung einführt.

Derivatisierungen im Rahmen der Erfindung sind beispielsweise eine Hydrochlorierung mit HCl in Dioxan, eine Umlagerung in Anwesenheit von N-Methylpyrrolidon unter Schutzgasatmosphäre und eine Cyclisierung mit Ameisensäure.

Die Verbindung der Formel (X) ist neu und kann aus den bekannten 8-Methoxy-2-hydroxymethylchroman durch Umsetzung mit HBr hergestellt werden [vgl hierzu EP 352 613].

Die Verbindungen der allgemeinen Formel (XI) sind bekannt.

Die Verbindungen der allgemeinen Formel (VI) sind neu und können beispielsweise hergestellt werden, indem man die oben aufgeführten Verbindungen der allgemeinen Formel (IV) mit Aminen der allgemeinen Formel (XII) 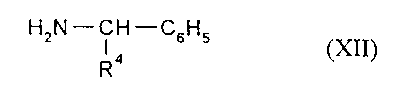 in welcher
- R⁴: die oben angegebene Bedeutung hat,
in Anwesenheit von Natriumjodid in einem Temperaturbereich von +50°C bis +150°C, vorzugsweise bei 100°C und Normaldruck umsetzt.

Die Amine der allgemeinen Formel (XII) sind bekannt.

Die Verbindungen der allgemeinen Formel (IV) sind als Species neu und können beispielsweise hergestellt werden, indem man die oben aufgeführten Verbindungen der allgemeinen Formel (VIII) mit Verbindungen der allgemeinen Formel (XIII)

R³-X (XIII)

in welcher
- R³: die oben angegebene Bedeutung hat
und
- X: für Halogen, vorzugsweise für Chlor steht,
in einem der oben aufgeführten Lösemitteln, vorzugsweise Pyridin, in einem Temperaturbereich von 0°C bis Raumtemperatur, vorzugsweise bei Raumtemperatur und Normaldruck umsetzt.

Die Verbindungen der allgemeinen Formel (XIII) sind bekannt.

Die Verbindungen der allgemeinen Formeln (III), (V) und (VII) sind an sich bekannt oder nach üblichen Methoden herstellbar.

Die erfindungsgemäßen Verbindungen können als Wirkstoffe in Arzneimitteln verwendet werden. Die erfindungsgemäßen Stoffe haben eine besonders hohe Affinität zu cerebralen 5-Hydroxy-tryptamin-Rezeptoren vom 5-HT₁-Typ.

Überraschenderweise zeigen die erfindungsgemäßen Verbindungen eine geringe Abhängigkeit in ihrer Metabolisierung von Leberenzymen des Typs CYP 2D6.

Die in der vorliegenden Erfindung beschriebenen Verbindungen stellen somit Wirkstoffe zur Bekämpfung von Krankheiten dar, die durch Störungen des serotoninergen Systems, insbesondere bei Involvierung von Rezeptoren, die hohe Affinität zu 5-Hydroxytryptamin besitzen (5-HT₁-Typ), gekennzeichnet sind. Sie eignen sich daher zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuß- und Suchtmittelaufnahme. Weiterhin sind sie geeignet zur Beseitigung kognitiver Defizite, zur Verbesserung von Lern- und Gedächtnisleistungen und zur Behandlung der Alzheimer'schen Krankheit.

Weiterhin eignen sich diese Wirkstoffe auch zur Modulierung des cardiovaskulären Systems. Sie greifen auch in die Regulation der cerebralen Durchblutung ein und stellen somit wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden. Auch eignen sie sich zur Bekämpfung von Störungen des Immunsystems.

### 1.) Affinität zum 5-HT₁-Rezeptor

In Tabelle A wird beispielhaft die hohe Affinität der erfindungsgemäßen Verbindungen zu 5-Hydroxytryptamin-Rezeptoren vom Subtyp 1 dargestellt. Bei den angegebenen Werten handelt es sich um Daten, die aus Rezeptorbindungsstudien mit Kalbs-Hippocampus-Membranpräparationen ermittelt wurden. Als radioaktiv markierter Ligand wurde hierzu ³H-Serotonin verwendet.

**Tabelle A**

| **Verbindung des Beispiels** | **K**_{**i**} **(nmol/l)** |
|---|---|
| 1 | 1.1 |
| 4 | 2.8 |
| 5 | 0.5 |

### 2.) Affinität zum 5-HT_{1A}-Rezeptor [W.U. Dompert et al., Naunyn-Schmiedeberg's Arch. Pharmacol. (1985), 328, 467-470].

Bei diesem Test wird die Bindung von ³H-Ipsapiron an 5-HT_{1A}-Rezeptoren in Kalbshippocampus-Membranen gemessen. Es wurde gefunden, daß die erfindungsgemäßen Verbindungen mit dem Radioliganden um die Bindung konkurrieren und diese hemmen.

**Tabelle B**

| **Verbindung des Beispiels** | **K**_{**i**} **(nmol/l)** |
|---|---|
| 2 | 0.5 |
| 3 | 1.8 |
| 6 | 0.9 |

Bei den Bindungstests unter 1) und 2) werden IC₅₀-Werte ermittelt, die angeben, bei welcher Konzentration an Testsubstanz 50% der Bindung des Radioliganden verdrängt wird. Unter Berücksichtigung der Dissoziationskonstanten und der Konzentration an Radioliganden werden daraus die Inhibitionskonstanten Kᵢ berechnet.

### 3.) Wirksamkeit in Tiermodellen

Tiermodell: permanente fokale cerebrale Ischämie ("middle cerebral artery occlusion" = MCA-O). Die MCA-Occlusion in Nagern ist ein breit akzeptiertes Tiermodell des Schlaganfalls. Literatur: Bederson, J.B. et al., Stroke, 17:472-476 (1986).

Um eine permanente fokale cerebrale Ischämie hervorzurufen wird in Ratten die linke Arterie cerebri media durch Elektrokoagulation okkludiert. Das resultierende Infarktvolumen in cortikalen (subcorticalen) Regionen, die von der mittleren cerebralen Arterie versorgt werden, wird als Maß für die Größe der Schlaganfall-induzierten neuronalen Schäden herangezogen. Substanzapplikation: Nach der Okklusion als kontinuierliche i.v. Infusion (4 Stunden) der Testsubstanz, direkt nach der Operation beginnend. Die Tiere werden 7 Tage nach der Operation zur Auswertung getötet.

### Results:

| **Beispiel** | **% Reduktion des Infarktvolumens** | **Dosis: mg/kg i.v.** |
|---|---|---|
| 2 | 36 | 0.001 |
| | 48 | 0.01 |
| | 32 | 0.1 |
| 6 | 41 | 0.001 |
| | 41 | 0.01 |
| | 38 | 0.1 |
| 3 | 37 | 0.001 |
| | 34 | 0.003 |
| | 47 | 0.01 |
| zum Vergleich: | (Zunahme um 21%) | 0.1 |
| Bsp. 86 aus | 15 | 1.0 |
| EP 352 613∗ | 1 | 3.0 |

| | | |
|---|---|---|
| ∗: MCA-O Maus, Substanzgabe als Bolusinjektionen direkt, 2 und 4 Stunden nach der Okklusion; Tötung nach 2 Tagen | | |

### 4.) Stabilität in humanen Lebermikrosomen mit und ohne Zugabe von Chinidin.

Um das Ausmaß der von Cytochrom P-450 (CYP) 2D6 abhängigen Biotransformation zu ermitteln, wurde der Phase I-Metabolismus von humanen Lebermikrosomen mit und ohne Zugabe von Chinidin, einem selektiven Inhibitor von CYP 2D6, untersucht. Die Flächen unter den Konzentrations-Zeitverläufen (AUCₙₒᵣₘ) und die Halbwertszeiten wurden bestimmt.
Im Falle der Verbindungen des Beispiel 2, Beispiel 3 und Beispiel 6 erhöht sich nach Zugabe von Chinidin die AUCₙₒᵣₘ und die Halbwertszeit um den Faktor 1.3 bis 2.0, während bei Beispiel 93.i aus EP 352 613 und Bsp. 4 aus EP 540 914 eine Erhöhung um den Faktor 4 bei AUCₙₒᵣₘ und Halbwertszeit zu beobachten ist.
Die erfindungsgemäßen Verbindungen zeigen vorteilhafterweise eine geringere Abhängigkeit vom Phase I-Metabolismus über das CYP 2D6-Enzym als bekannte Chromane.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Ausgangsverbindungen

### Beispiel I

(-)-8-Hydroxy-2-hydroxymethylchroman 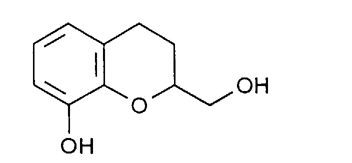 135.5 g (0.7 Mol) (-)-2-Hydroxymethyl-8-methoxychroman werden 20 Stunden in 0.7 1 48 %ige wäßriger HBr-Lösung erhitzt. Nach Abkühlen und Verdünnen mit 1.21 Eiswasser wird 30 Minuten gerührt, und der ausgefallenen Niederschlag abgesaugt. Waschen mit Eiswasser und Trocknen über Phosphorpentoxid liefert 109.5 g (87 %) der Titelverbindung, Schmp. 131-132°C.
α₂₈₉²⁰ = -133.8 (c=0.7, Methanol)

### Beispiel II

2-Hydroxymethyl-8-isopropoxy-chroman 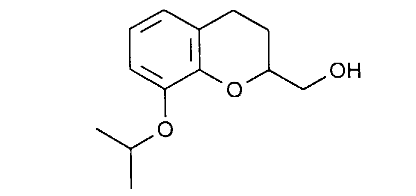 4.5 g (25 mMol) 8-Hydroxy-2-hydroxymethyl-chroman, 4.6 g (27 mMol) 2-Iod-propan und 5.2 g (37.5 mMol) gepulvertes Kaliumcarbonat in 50 ml Dimethylformamid werden 40 Stunden auf 60°C erhitzt. Nach Zugabe weiterer 0.9 g Iod-propan wird 24 Stunden auf 80°C und anschließend weitere 24 Stunden auf 95°C erhitzt. Nach Abkühlen wird zwischen Toluol/Wasser verteilt und über Celite® filtriert. Die organische Phase wird getrocknet (Magnesiumsulfat) und eingeengt. Nach Flashchromatographie (Kieselgel; Elution mit Toluol/Essigester-Gradienten 3:1 - 2:1) erhält man 7 g Rohprodukt, das durch Chromatographie auf Kieselgel (Gradient Toluol/Essigester 1:0 - 8:1) gereinigt wird. Ausbeute: 2.9 g (52%) Öl. R_{F} (Kieselgel, Toluol/Essigester 1:1): 0.4

### Beispiel III

(-)-2-Hydroxymethyl-8-isopropoxy-chroman

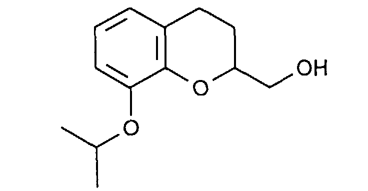

In Analogie zur Vorschrift des Beispiels II erhält man die Titelverbindung aus der Verbindung des Beispiels I.
α₂₈₉²⁰=-85 [c=0.5, CHCl₃]

### Beispiel IV

2-Hydroxymethyl-8-(2-methyl-propen-2-yloxy)chroman 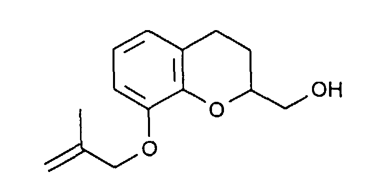 0.9 g (5 mMol) 8-Hydroxy-2-hydroxymethyl-chroman, 0.5 g (5.5 mMol) 3-Chlor-2-methylpropen, 0.02 g Natriumiodid und 1.0 g (7.5 mMol) gepulvertes Kaliumcarbonat werden in 10 ml Dimethylformamid 3 Stunden auf 90°C erhitzt. Nach Abkühlen wird zwischen Wasser und Toluol verteilt. Die Toluolphase wird durch Flashchromatographie über Kieselgel (Toluol/Essigester) gereinigt. Man erhält so 1.3 g (100 % = 1.2 g) Rohprodukt, das weiter umgesetzt wird.
R_{F} (Kieselgel, Toluol/Essigester 1:1): 0.45

### Beispiel V

(-)-2-Hydroxymethyl-8-(2-methyl-propen-2-yloxy)chroman

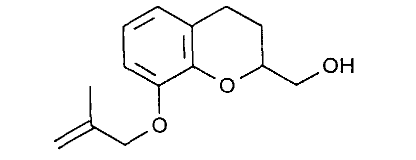

In Analogie zur Vorschrift des Beispiels IV erhält man die Titelverbindung aus Beispiel I.
α₂₈₉²⁰= -87 [c=0.5, CHCl₃]

### Beispiel VI

2-Hydroxymethyl-8-(2-chlor-2-methyl-propoxy)chroman 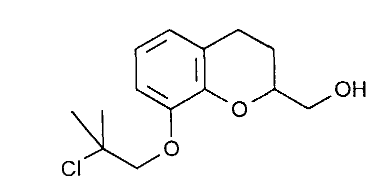 12.5 g (53.4 mMol) der Verbindung aus Beispiel IV werden in 4N Salzsäure in Dioxan 6 Stunden auf 55°C erhitzt. Nach Einengen wird der Rückstand durch Flashchromatographie auf Kieselgel (Toluol-Essigester 1:0 - 20:1) gereinigt. Man erhält so 4.0 g der Titelverbindung als Öl. Mit Toluol/Essigester werden 4.0 g (42 %) unumgesetztes Ausgangsmaterial zurückgewonnen.
¹H-NMR (CDCl₃, δ-Werte in ppm): 1.7 (s; 6H), 1.7 - 2.1 (m, 2H), 2.6 (bs; 1H), 2.7 - 3.0 (m; 2H), 3.7 - 3.9 (m, 2H), 4.0 (s; 2H), 4.05 - 4.15 (m; 1H) 6.65-6.8 (m, 3H)

### Beispiel VII

(-)-2-Hydroxymethyl-8-(2-chlor-2-methyl-propoxy)chroman 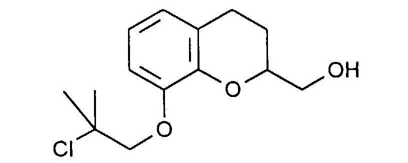 Analog zu Beispiel VI erhältlich aus Bsp. V; α₂₈₉²⁰= -70 [c=0.4, CHCl₃]

### Beispiel VIII

8-Hydroxy-2-hydroxymethyl-7-(2-methyl-propen-2-yl)chroman 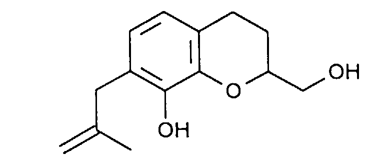 1.3 g (5 mMol) Verbindung aus Beispiel IV werden in 4 ml N-Methylpyrrolidon gelöst und unter Argon 4 Stunden auf 210°C erhitzt. Nach Abkühlen wird über Kieselgel (Toluol/Essigester-Gemische) filtriert. Die Eluate werden eingeengt und zwischen Diethylether und Wasser verteilt. Nach Trocknen (Magnesiumsulfat) und Einengen erhält man 1.0 g (77 %) Rohprodukt als Öl, das direkt weiter umgesetzt wird.

### Beispiel IX

(-)-8-Hydroxy-2-hydroxymethyl-7-(2-methyl-propen-2-yl)chroman

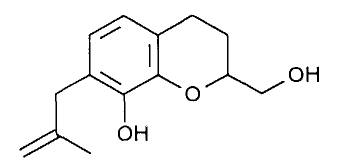

In Analogie zur Vorschrift des Beispiels VIII erhält man die Titelverbindung aus Beispiel V.
α₂₈₉²⁰= -88 [c=1, CHCl₃]

### Beispiel X

2,2-Dimethyl-3,6,7,8-tetrahydro-2H-1,9-dioxa-cyclopenta[a]naphthalen-8-yl-methanol 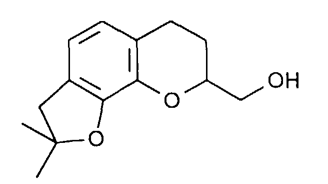 1.0 g (4 mMol) Verbindung aus Beispiel VIII wird in 3 ml Ameisensäure 2 Stunden zum Rückfluß erhitzt. Nach dem Einengen wird mit 10 ml 20 % Kaliumhydroxid in Wasser versetzt und 1 Stunde zum Rückfluß erhitzt. Das Gemisch wird nach Abkühlen mit Salzsäure angesäuert und mit Essigester extrahiert. Die organische Phase wird getrocknet (Magnesiumsulfat) und eingeengt. Flashchromatographie über Kieselgel (Elution mit Toluol/Essigester-Gradienten 1:0 - 3:1) ergibt 0.73 g (78%) der Titelverbindung als Sirup.
¹H-NMR (CDCl₃, δ-Werte in ppm): 1.5 (s; 6H), 1.9 - 2.1 (m; 2H), 2.7 - 2.9 (m; 2H), 3.0 ("s"; 2H), 3.7 - 4.0 (m; 2H), 4.1 - 4.2 (m; 1H), 6.4 - 6.7 (AB-System; 2H)

### Beispiel XI

(-)-2,2-Dimethyl-3,6,7,8-tetrahydro-2H-1,9-dioxa-cyclopenta[a]naphthalen-8-yl-methanol

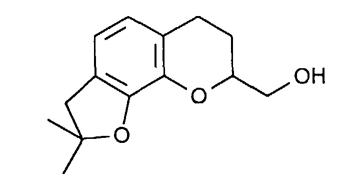

In Analogie zur Vorschrift des Beispiels X erhält man die Titelverbindung aus Beispiel IX.
α₂₈₉²⁰= -87 [c=0.5, CHCl₃]

### Beispiel XII

8-(2-Chlor-2-methyl-propoxy)-2-(phthalimidomethyl)chroman

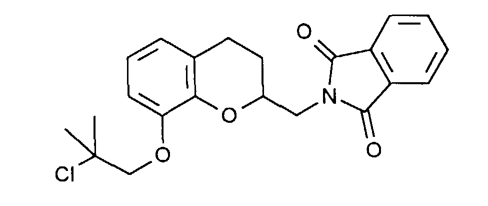

Zu 4.0 g (17 mMol) Verbindung aus Beispiel VI, 4.9 g (18.5 mMol) Triphenylphosphan und 2.7 g (18.5 mMol) Phthalimid in 40 ml Tetrahydrofuran werden langsam bei Raumtemperatur unter Lichtausschluß 3.3 g (18.5 mMol) Azodicarbonsäurediethylester in 15 ml Tetrahydrofuran zugetropft. Nach 1 Stunde bei Raumtemperatur wird eingeengt und der Rückstand durch Flashchromatographie gereinigt (Cyclohexan/Essigester-Gradienten 1:0 - 20:1).

R_{F} (Toluol/Essigester 3:1): 0.27

Die in Tabelle 1 aufgeführten Verbindungen können in Analogie zu Beispiel XII erhalten werden.

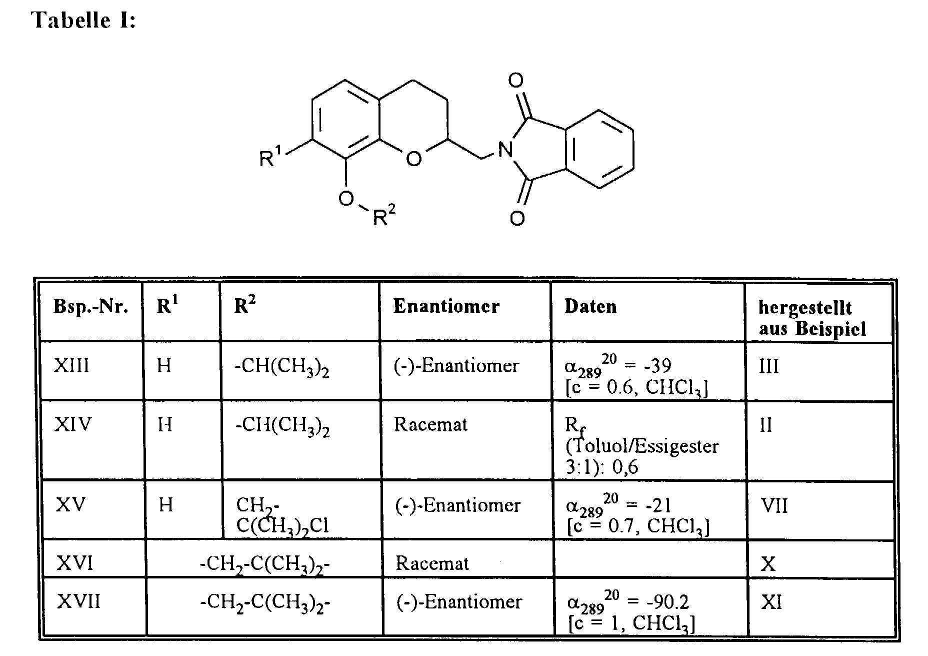

### Beispiel XVIII

2-Aminomethyl-8-(2-chlor-2-methyl-propoxy)chroman 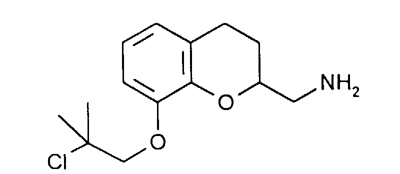 5.8 g (14.5 mMol) der Verbindung aus Beispiel XII werden mit 13.4 g (220 mMol) Aminoethanol 30 Minuten auf 80°C erhitzt. Nach Abkühlen wird mit Wasser verdünnt und mit Essigester extrahiert. Trocknen der organischen Phase und Einengen liefert ein Rohprodukt, das durch Flashchromatographie auf Kieselgel (Elution mit Toluol-Isopopanol-Gradienten 1:0 bis 1:1, Zusatz von 1 % Triethylamin) gereinigt wird. Man erhält so 3.35 g (86 %) Titelverbindung als Öl.
R_{F} (Dichlormethan/Methanol 10:1): 0.15

Die in Tabelle 2 aufgeführten Verbindungen können in Analogie zu Beispiel XVIII erhalten werden.

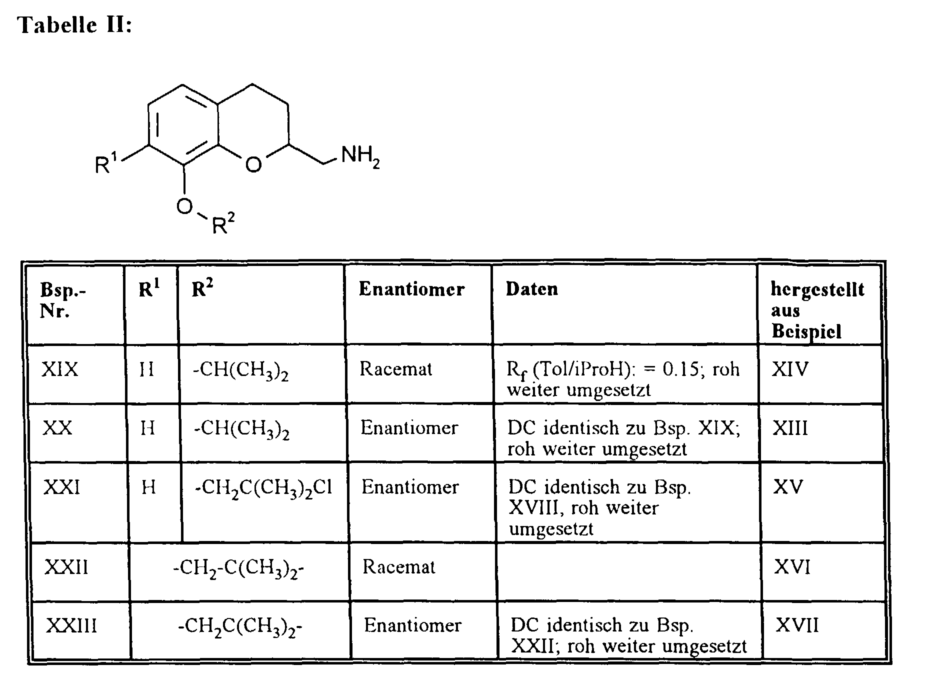

### Beispiel XXIV

(-)-8-Isopropoxy-2-mesyloxymethyl-chroman

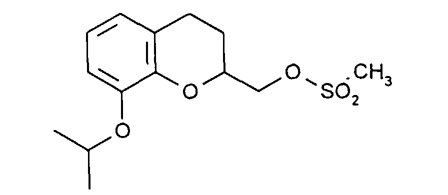

Zu 114 g (0.51 Mol) der Verbindung aus Beispiel III in 95 g Pyridin werden bei Raumtemperatur 68 g (0.6 Mol) Methansulfonsäurechlorid getropft. Nach 18 Stunden Rühren bei Raumtemperatur wird mit 700 ml Wasser verdünnt und mit Dichlormethan extrahiert. Filtration über Kieselgel und Einengen ergibt 150 g Rohprodukt, das durch Kristallisation aus 1.5 1 Cyclohexan/Toluol-Gemisch 3:1 gereinigt wird. Die Mutterlauge wird nach Einengen aus Cyclohexan umkristallisiert. Man erhält so insgesamt 112g Titelverbindung als farblosen Feststoff, Schmp. 77-78°C.
α₂₈₉²⁰= -56.2 [c=0.9, CH₃OH]

### Beispiel XXV

(-)-2-Benzylaminomethyl-8-isopropoxy-chroman 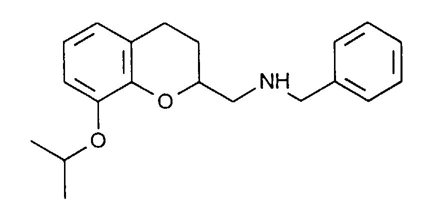 112 g (0.37 Mol) der Verbindung aus Beispiel XXIV, 200 g (1.87 Mol) Benzylamin und 3.0 g (0.02 Mol) Natriumiodid werden 5 Stunden auf 100°C erhitzt. Nach Abkühlen wird von Feststoff abgetrennt und die organische Phase 2x mit je 2.5 l Wasser gewaschen. Das verbleibende Öl wird mit 1 Essigester aufgenommen. Waschen der Essigesterphase mit Wasser und gesättigter Kochsalzlösung sowie anschließendes Trocknen und Einenengen ergibt 114.5 g (quant.) der Titelverbindung (HPLC-Reinheit: 93 %) als Öl, die in die nächste Stufe eingesetzt wird.
α₂₈₉²⁰ = -104 [c=0.5, CH₃OH]

### Beispiel XXVI

(-)-2-(N-Benzyl-N-(4-(1,1-dioxido-3-oxo-2,3-dihydro-benzisothiazol-2-yl)-2-butinyl)- aminomethyl)-8-isopropoxy-chroman Hydrochlorid 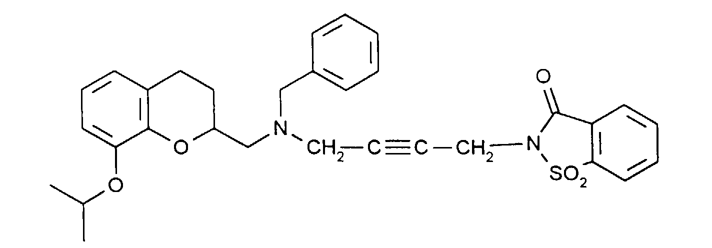 114 g (0.37 Mol) der Verbindung aus Beispiel XXV und 13.5 g (0.45 Mol) Paraformaldehyd in 1 1 Dioxan werden mit 4 g Kupfer(II)acetat versetzt und auf 50°C erwärmt Bei dieser Temperatur werden 81 g (0.37 Mol) Propargylsaccharin zugesetzt. Nach 2 Stunden Rühren bei 80°C wird eingeengt und der Rückstand zwischen Toluol/Wasser unter Zusatz von Tonsil verteilt. Nach Filtration des Gemisches über Celite® wird die organische Phase abgetrennt und durch Flashchromatographie über Kieselgel (Toluol/Essigester 10:1) gereinigt. Fällung des Hydrochlorids aus Ether mit etherischer Salzsäure ergibt 226 g Rohprodukt. Dieses Produkt wird nach Freisetzung der freien Base mit Natriumhydrogencarbonat durch Chromatographie auf Kieselgel (Elution mit Toluol/Essigester 20:1) gereinigt. Die Produktfraktionen werden mit etherischer Salzsäure behandelt. Man erhält so 139 g (65 %) Titelverbindung als Feststoff, Schmp. 106-109°C.
α₂₈₉²⁰ = - 64,1 [c=0.8, CH₃OH]

### Herstellungsbeispiele

### Beispiel 1

2-(N-(4-(1,1-Dioxido-3-oxo-2,3-dihydro-benzisothiazol-2-yl)butyl)amino)methyl-8-isopropoxy-chroman Oxalat 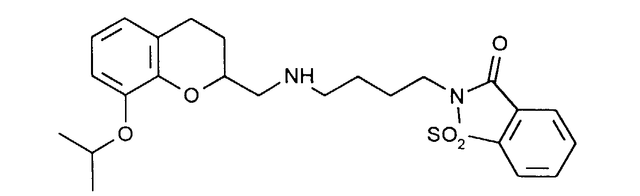 2.7 g (12 mMol) der Verbindung aus Beispiel XIX werden in 35 ml Dimethylformamid gelöst und mit 3.4 g (10.5 mMol) 4-Brombutylsaccharin sowie 2.5 g (25 mMol) Triethylamin versetzt. Nach Zugabe von 50 mg Kaliumiodid wird 15 Stunden bei 50°C gerührt. Das Reaktionsgemisch wird mit Wasser verdünnt und mehrmals mit Toluol extrahiert. Die organische Phase wird getrocknet und eingeengt. Zweimalige Chromatographie mit Toluol-Essigestergradienten ergibt 3.0 g Rohprodukt (freie Base). Mit Oxalsäure wird in Ethanol das Oxalat gefällt, das aus Wasser umkristallisiert wird. Man erhält so 2.4 g Oxalat, Schmp. 179-180°C.

| Elementaranalyse: C₂₄ H₃₀ N₂ O₅ S x C₂ H₂ O₄ x 0.5 H₂O | | | | |
|---|---|---|---|---|
| ber. | C: 56.0 | H: 5.9 | N: 5.0 | O: 27.3 |
| gef. | C: 55.8 | H: 6.1 | N: 5.0 | O: 27.1 |

Die in Tabelle 1 aufgeführten Verbindungen können in Analogie zu Beispiel 1 erhalten werden.

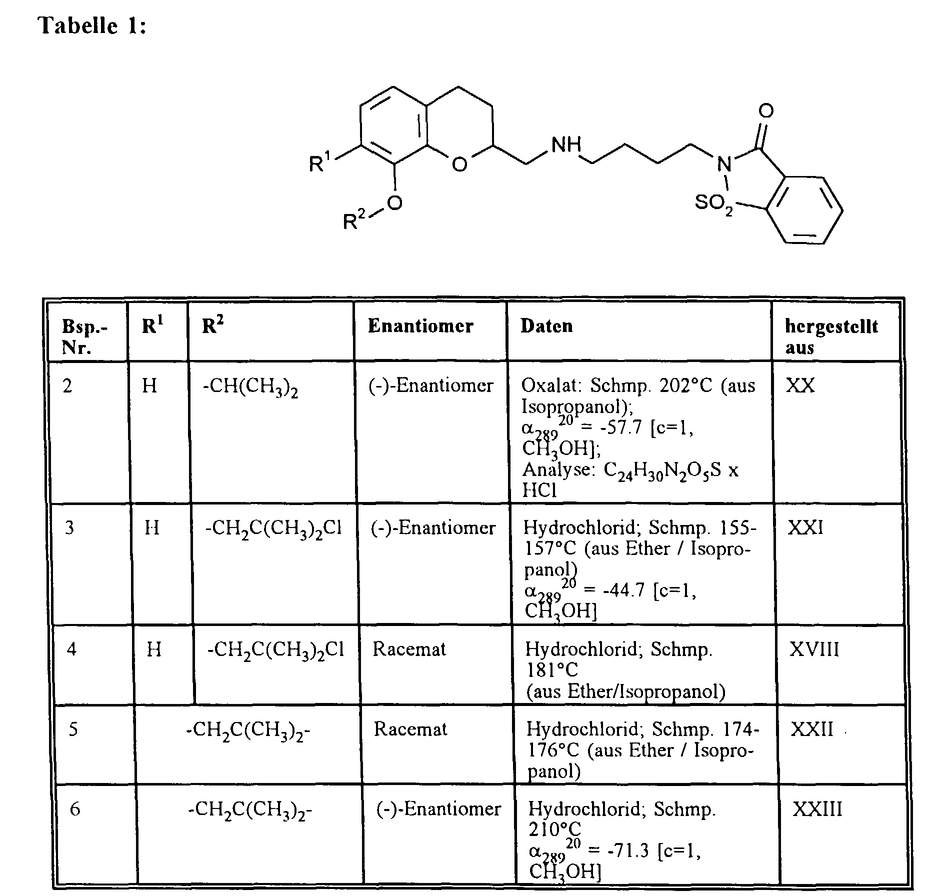

### Beispiel 7

(-)-2-(N-(4-(1,1-Dioxido-3-oxo-2,3-dihydro-benzisothiazol-2-yl)butyl)amino)methyl-8-isopropoxy-chroman Hydrochlorid 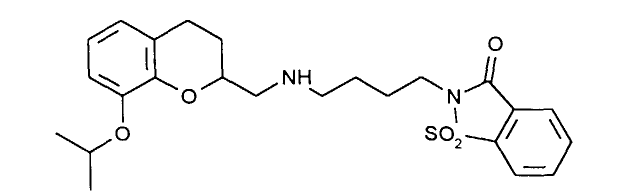 120 g (0.21 mMol) der Verbindung aus Beispiel XXVI in 1.4 l Methanol werden mit 400 ml konz. Salzsäure und 20 g 10 % Palladium auf Aktivkohle versetzt.

Nach 4 Stunden Hydrieren bei Normaldruck und 20°C wird vom Katalysator abfiltriert und eingeengt. Der Rückstand wird 2x mit Toluol eingeengt und mit 400 ml Essigester gelöst. Zusatz von 800 ml Diethylether und 18 h Rühren bei Raumtemperatur ergibt nach Absaugen und Trocknen im Vakkum 90.5 g Feststoff. Umkristallisation aus 1 l Acetonitril und Waschen der Kristalle mit Dieethylether ergibt 70.8 g (69 %) Titelverbindung als farblose Kristalle, Schmp. 153-154°C.
α₂₈₉²⁰= -65.9 [c=0.6, CH₃OH]

| Elementaranalyse: C₂₄ H₃₀ N₂ O₅ S x HCl | | | | | | |
|---|---|---|---|---|---|---|
| ber. | C: 58.2 | H: 6.3 | N: 5.7 | O: 16.2 | Cl: 7.2 | S: 6.5 |
| gef. | C: 58.0 | H: 6.3 | N: 5.7 | O: 16.2 | Cl: 7.1 | S: 6.3 |

## Patentansprüche

1. Benzisothiazolyl-substituierte Aminomethylchromane der allgemeinen Formel (I), 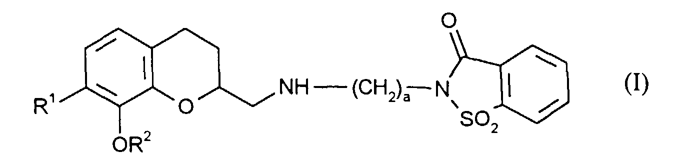 in welcher
R¹ für Wasserstoff steht,
und
R² für einen Rest der Formel -CH(CH₃)₂ oder -CH₂-C(CH₃)₂-Cl steht,
oder
R¹ und R² gemeinsam einen Rest der Formel 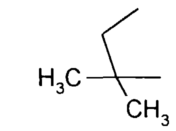 bilden,
und
a für eine Zahl 3, 4 oder 5 steht,
gegebenenfalls in einer isomeren Form und deren Salze.

2. Benzisothiazolyl-substituierte Aminomethylchromane der Formel nach Anspruch 1,
in welcher
R¹ für Wasserstoff steht,
und
R² für einen Rest der Formel -CH(CH₃)₂ oder -CH₂-C(CH₃)₂-Cl steht,
oder
R¹ und R² gemeinsam einen Rest der Formel 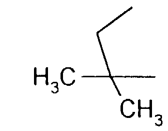 bilden,
und
a für eine Zahl 3 oder 4 steht,
gegebenenfalls in einer isomeren Form und deren Salze.

3. Benzisothiazolyl-substituierte Aminomethylchromane der Formel nach Anspruch 1,
in welcher
R¹ für Wasserstoff steht,
und
R² für einen Rest der Formel -CH(CH₃)₂ oder -CH₂-C(CH₃)₂-Cl steht,
oder
R¹ und R² gemeinsam einen Rest der Formel 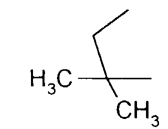 bilden,
und
a für die Zahl 4 steht,
gegebenenfalls in einer isomeren Form und deren Salze.

4. Benzisothiazolyl-substituierte Aminomethylchromane der Ansprüche 1 bis 3 zur therapeutischen Anwendung.

5. Verfahren zur Herstellung von Benzisothiazolyl-substituierten Aminomethylchromanen nach Ansprüchen 1 bis 3, dadurch gekennzeichnet, daß man
[A] Amine der allgemeinen Formel (II) 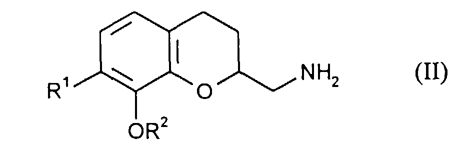 in welcher
R¹ und R² die oben angegebene Bedeutung haben,
mit Verbindungen der allgemeinen Formel (III) 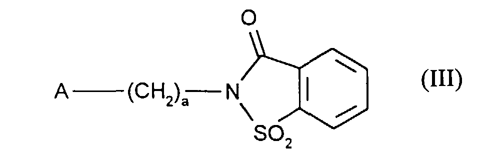 in welcher
A für eine typische Abgangsgruppe wie Chlor, Brom, Iod, Tosylat, Mesylat oder für die Gruppe -OSO₂CF₃ steht,
und
a die oben angegebene Bedeutung hat,
in inerten Lösemitteln, gegebenenfalls in Anwesenheit einer Base umsetzt,
oder
[B] Verbindungen der allgemeinen Formel (IV) 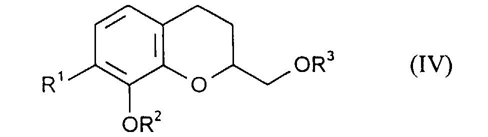 in welcher
R¹ und R² die oben angegebene Bedeutung haben
und
R³ für einen Rest der Formel -SO₂CF₃, -SO₂-CH₃ oder Tosylat steht,
mit Aminen der allgemeinen Formel (V) 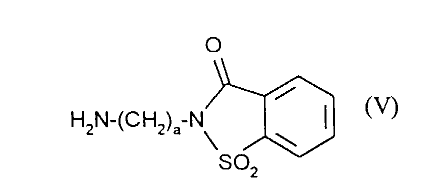 in welcher
a die oben angegebene Bedeutung hat,
in inerten Lösemitteln, gegebenenfalls in Anwesenheit einer Base und/oder eines Katalysators umsetzt,
oder
[C] Verbindungen der allgemeinen Formel (VI) 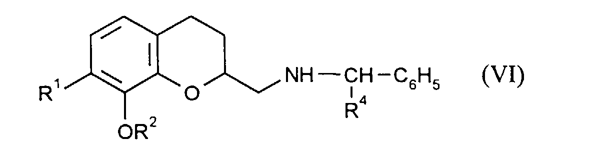 in welcher
R¹ und R² die oben angegebene Bedeutung haben,
und
R⁴ für Wasserstoff oder Methyl steht,
zunächst entweder mit Formaldehyd oder Formaldehydderivaten und Verbindungen der allgemeinen Formel (VII) 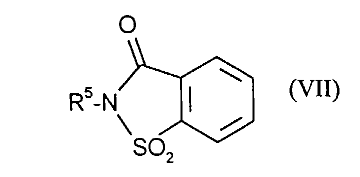 in welcher
R⁵ für einen Rest der Formel
HC≡C-(CH₂)_{b}-
steht,
worin
b eine Zahl 0, 1 oder 2 bedeutet,
in einer Mannich-analogen Reaktion umsetzt und dann hydriert.

6. Arzneimittel enthaltend mindestens ein Benzisothiazolyl-substituiertes Aminomethylchroman nach Ansprüchen 1 bis 3 sowie übliche Hilfs- und Zusatzstoffe.

7. Arzneimittel nach Anspruch 6 zur Behandlung von Schlaganfall.

8. Verfahren zur Herstellung von Arzneimitteln nach Ansprüchen 6 und 7, dadurch gekennzeichnet, daß man die Wirkstoffe mit üblichen Hilfs- und Zusatzstoffe in eine geeignete Applikationsform überführt.

9. Verwendung von Benzisothiazolyl-substituierten Aminomethylchromanen nach Ansprüchen 1 bis 3 zur Herstellung von Arzneimitteln.

10. Verwendung nach Anspruch 9, dadurch gekennzeichnet, daß die Arzneimittel zur Behandlung von Schlaganfall sind.

## Claims

1. Benzisothiazolyl-substituted aminomethylchromans of the general formula (I) 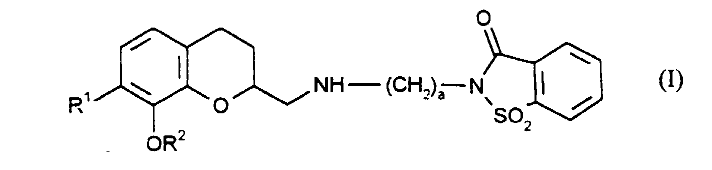 in which
R¹ represents hydrogen,
and
R² represents a radical of the formula -CH(CH₃)₂ or -CH₂-C(CH₃)₂-Cl,
or
R¹ and R² together form a radical of the formula 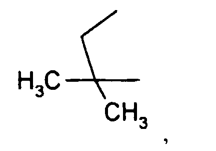
and
a represents a number 3, 4 or 5,
if appropriate in an isomeric form, and their salts.

2. Benzisothiazolyl-substituted aminomethylchromans of the formula according to Claim 1,
in which
R¹ represents hydrogen,
and
R² represents a radical of the formula -CH(CH₃)₂ or -CH₂-C(CH₃)₂-Cl,
or
R¹ and R² together form a radical of the formula 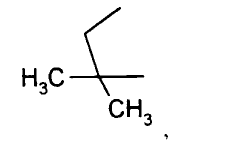
and
a represents a number 3 or 4,
if appropriate in an isomeric form, and their salts.

3. Benzisothiazolyl-substituted aminomethylchromans of the formula according to Claim 1,
in which
R¹ represents hydrogen,
and
R² represents a radical of the formula -CH(CH₃)₂ or -CH₂-C(CH₃)₂-Cl,
or
R¹ and R² together form a radical of the formula 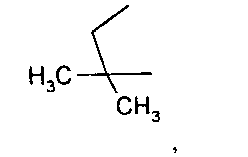
and
a represents the number 4,
if appropriate in an isomeric form, and their salts.

4. Benzisothiazolyl-substituted aminomethylchromans of Claims 1 to 3 for therapeutic use.

5. Process for the preparation of benzisothiazolyl-substituted aminomethylchromans according to Claims I to 3, characterized in that
[A] amines of the general formula (II) 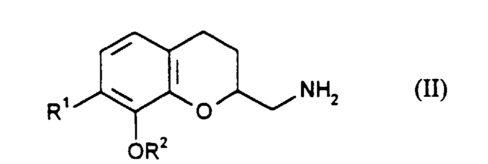 in which
R¹ and R² have the meaning indicated above,
are reacted with compounds of the general formula (III) 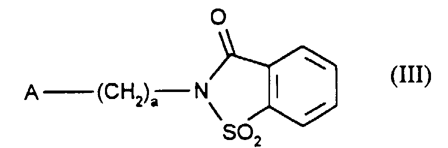 in which
A represents a typical leaving group such as chlorine, bromine, iodine, tosylate, mesylate or the group -OSO₂CF₃,
and
a has the meaning indicated above,
in inert solvents, if appropriate in the presence of a base,
or
[B] compounds of the general formula (IV) 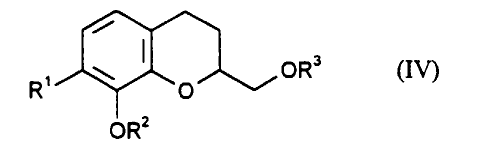 in which
R¹ and R² have the meaning indicated above
and
R³ represents a radical of the formula -SO₂CF₃, -SO₂-CH₃ or tosylate,
are reacted with amines of the general formula (V) 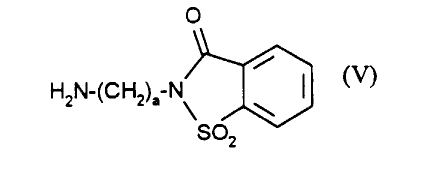 in which
a has the meaning indicated above,
in inert solvents, if appropriate in the presence of a base and/or of a catalyst,
or
[C] compounds of the general formula (VI) 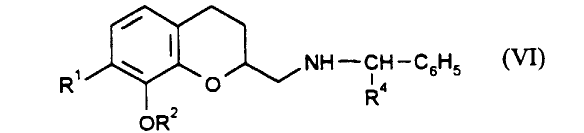 in which
R¹ and R² have the meaning indicated above,
and
R⁴ represents hydrogen or methyl,
are reacted first either with formaldehyde or formaldehyde derivatives and compounds of the general formula (VII) 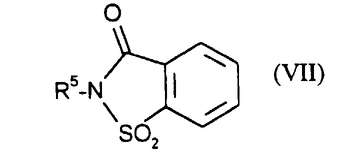 in which
R⁵ represents a radical of the formula
HC≡(CH₂)_{b}-
in which
b denotes a number 0, 1 or 2,
in a Mannich-analogous reaction and then hydrogenated.

6. Medicaments containing at least one benzisothiazolyl-substituted aminomethylchroman according to Claims 1 to 3 and customary auxiliaries and additives.

7. Medicaments according to Claim 6 for the treatment of stroke.

8. Process for the production of medicaments according to Claims 6 and 7, characterized in that the active compounds are converted into a suitable administration form using customary auxiliaries and additives.

9. Use of benzisothiazolyl-substituted aminomethylchromans according to Claims 1 to 3 for the production of medicaments.

10. Use according to Claim 9, characterized in that the medicaments are for the treatment of stroke.

## Revendications

1. Aminométhylchromanes à substituant benzisothiazolyle, de formule générale (I) 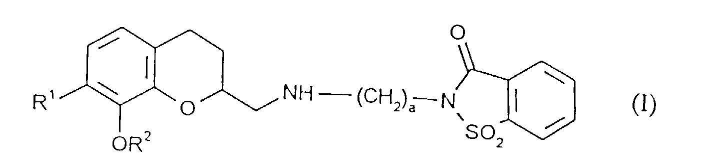 dans laquelle
R¹ est l'hydrogène,
et
R² représente un reste de formule -CH(CH₃)₂ ou -CH₂-C(CH₃)₂-Cl,
ou bien
R¹ et R² forment ensemble un reste de formule 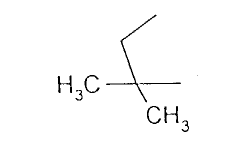
et
a représente le nombre 3, 4 ou 5,
le cas échéant sous une forme isomère, et leurs sels.

2. Aminométhylchromanes à substituant benzisothiazolyle de formule suivant la revendication 1, dans laquelle
R¹ est l'hydrogène,
et
R² représente un reste de formule -CH(CH₃)₂ ou -CH₂-C(CH₃)₂-Cl,
ou bien
R¹ et R² forment ensemble un reste de formule 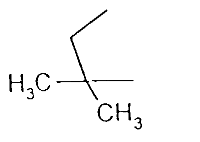
et
a représente le nombre 3 ou 4,
le cas échant sous une forme isomère, et leurs sels.

3. Aminométhylchromanes à substituant benzisothiazolyle de formule suivant la revendication 1,
dans laquelle
R¹ est l'hydrogène,
et
R² représente un reste de formule -CH(CH₃)₂ ou -CH₂-C(CH₃)₂-Cl,
ou bien
R¹ et R² forment ensemble un reste de formule 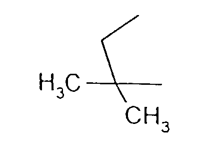
et
a représente le nombre 4,
le cas échéant sous une forme isomère, et leurs sels.

4. Aminométhylchromanes à substituant benzisothiazolyle suivant les revendications 1 à 3, destinés à une application thérapeutique.

5. Procédé de production d'aminométhylchromanes à substituant benzisothiazolyle suivant les revendications 1 à 3, caractérisé en ce que
[A] on fait réagir des amines de formule générale (II) 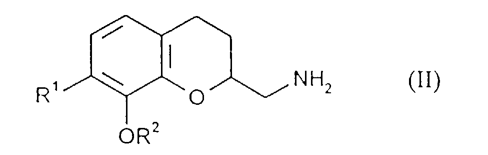 dans laquelle
R¹ et R² ont la définition indiquée ci-dessus,
avec des composés de formule générale (III) 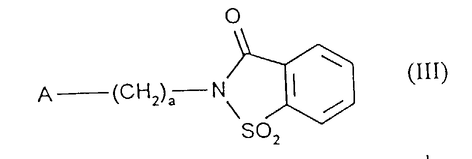 dans laquelle
A représente un groupe partant typique tel que chlore, brome, iode, tosylate, mésylate ou le groupe -OSO₂CF₃,
et
a a la définition indiquée ci-dessus,
dans des solvants inertes, éventuellement en présence d'une base,
ou bien
[B] on fait réagir des composés de formule générale (IV) 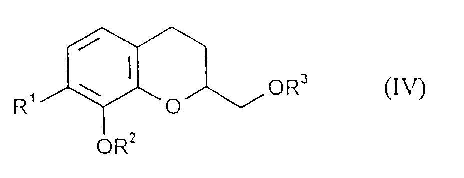 dans laquelle
R¹ et R² ont la définition indiquée ci-dessus,
et
R³ représente un reste de formule -SO₂CF₃, -SO₂-CH₃ ou tosylate,
avec des amines de formule générale (V) 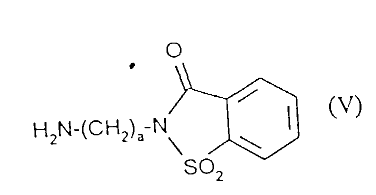 dans laquelle
a a la définition indiquée ci-dessus,
dans des solvants inertes, éventuellement en présence d'une base et/ou d'un catalyseur,
ou bien
[C) on fait tout d'abord réagir des composés de formule générale (VI) 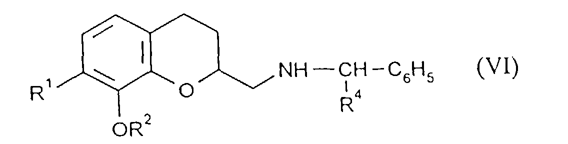 dans laquelle
R¹ et R² ont la définition indiquée ci-dessus,
et
R⁴ est l'hydrogène ou le groupe méthyle,
avec le formaldéhyde ou des dérivés de formaldéhyde et des composés de formule générale (VII) 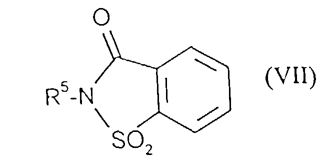 dans laquelle
R⁵ représente un reste de formule
HC≡C-(CH₂)_{b}-
où
b représente le nombre 0, 1 ou 2,
dans une réaction analogue à une réaction de Mannich, et on effectue ensuite une hydrogénation.

6. Médicaments contenant au moins un aminométhylchromane à substituant benzisothiazolyle suivant les revendications 1 à 3 ainsi que des substances auxiliaires et des additifs classiques.

7. Médicaments suivant la revendication 6, destinés au traitement de l'apoplexie cérébrale.

8. Procédé de préparation de médicaments suivant les revendications 6 et 7, caractérisé en ce qu'on fait prendre aux substances actives, avec des substances auxiliaires et des additifs classiques, une forme d'administration appropriée.

9. Utilisation d'aminométhylchromanes à substituant benzisothiazolyle suivant les revendications 1 à 3 pour la préparation de médicaments.

10. Utilisation suivant la revendication 9, caractérisée en ce que les médicaments sont destinés au traitement de l'apoplexie cérébrale.
